(19) Europäisches Patentamt
European Patent Office
Office européen des brevets

(11) Veröffentlichungsnummer : **0 381 040 B1**

(12) # EUROPÄISCHE PATENTSCHRIFT

(45) Veröffentlichungstag der Patentschrift : **31.03.93 Patentblatt 93/13**

(51) Int. Cl.$^5$ : **C07C 47/02,** C07C 45/50

(21) Anmeldenummer : **90101449.8**

(22) Anmeldetag : **25.01.90**

(54) **Verfahren zur Herstellung von 2,2-Dialkylpropionaldehyden.**

(30) Priorität : **01.02.89 DE 3902892**

(43) Veröffentlichungstag der Anmeldung : **08.08.90 Patentblatt 90/32**

(45) Bekanntmachung des Hinweises auf die Patenterteilung : **31.03.93 Patentblatt 93/13**

(84) Benannte Vertragsstaaten : **BE DE ES FR GB IT NL**

(56) Entgegenhaltungen :
**EP-A- 0 096 986**
**GB-A- 1 247 125**

(56) Entgegenhaltungen :
**CHEMICAL ABSTRACTS, Band 70, Nr. 24, 1969, Seite 278, Zusammenfassung Nr. 114537f, Columbus, Ohio, US; K.A. ALEKSEEVA et al.:** "**Composition of isobutylene hydroformylation products**"

(73) Patentinhaber : **BASF Aktiengesellschaft**
**Carl-Bosch-Strasse 38**
**W-6700 Ludwigshafen (DE)**

(72) Erfinder : **Bertleff, Werner, Dr.**
**Franz-Marc-Strasse 12**
**6806 Viernheim (DE)**
Erfinder : **Koeffer, Dieter, Dr.**
**Zinkgraefstrasse 1**
**W-6940 Weinheim (DE)**

EP 0 381 040 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von 2,2-Dialkylpropionaldehyden der allgemeinen Formel I

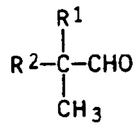 I

in der $R^1$ und $R^2$ für $C_1$ bis $C_4$-Alkylgruppen stehen, durch Hydroformylierung von 1,1-Dialkylethylenen der allgemeinen Formel II

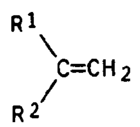 II

mit Cobaltcarbonyl-Komplexen. 2,2-Dialkylpropionaldehyde sind wichtige Zwischenprodukte für zahlreiche organische Synthesen, zum Beispiel für die Herstellung von Pharmazeutika.

Die Literaturstelle Chemical Engineering Progress, Vol. 62, Nr. 4, S. 74-78, 1966, betrifft die Hydroformylierung von Isobuten, wobei neben dem Hauptprodukt 3-Methylbutyraldehyd wenig Pivalaldehyd (2,2-Dimethylpropionaldehyd) gebildet wird. Die Ausbeute an Pivalaldehyd beträgt im günstigsten Fall 5,2 %, erzielt bei einem Druck von ca. 415 bar, einer Temperatur von 200 bis 220°C und einer Konzentration des Cobaltcarbonyl-Katalysators von 0,26 bis 0,28 Gew.% Cobalt, bezogen auf Isobuten, sowie mit Methanol oder Wasser als Lösungsmittel. In Wasser ist der Anteil an Pivalaldehyd geringer als in Methanol.

Nach der Literaturstelle Zhurnal Prikladnoi Khimii, Vol. 41, Nr. 10, S. 2275-2281, 1968, erhält man bei der Hydroformylierung von Isobuten mit Cobaltcarbonyl-Komplexen ebenfalls 3-Methylbutyraldehyd als Hauptprodukt. Daneben entsteht Pivalaldehyd in einer Ausbeute von 5 bis 6 %.

Auch in der Literaturstelle Karbonilirovanie Nenasyshchennykh Uglevodorodov, 1968, S. 75-80 (Chemical Abstracts Referat: Vol. 70, 114537f, 1969) führt die Hydroformylierung von Isobuten in Toluol mit Cobaltcarbonyl-Komplexen bei ca. 290 bar, 175 bis 190°C und einer Konzentration des Cobaltcarbonyl-Komplexes von 0,65 Gew.% Cobalt überwiegend zu 3-Methylbutyraldehyd und zu Pivalaldehyd nur in einer Ausbeute von ca. 5 %.

Aufgabe der vorliegenden Erfindung war es daher, den Anteil an 2,2-Dialkylpropionaldehyden I bei der Hydroformylierung von 1,1-Dialkylethylenen II zu erhöhen.

Demgemäß wurde ein Verfahren zur Herstellung von 2,2-Dialkylpropionaldehyden I durch Hydroformylierung von 1,1-Dialkylethylenen II mit Cobaltcarbonyl-Komplexen gefunden, das dadurch gekennzeichnet ist, daß man

(a) die Umsetzung in Gegenwart von wasserhaltigen Lösungsmitteln durchführt, in denen Wasser löslich oder zumindest teilweise löslich ist, wobei der Wassergehalt des Reaktionsgemisches zwischen 0,1 und 20 Gew.% liegt, oder

(b) die Umsetzung in Gegenwart eines Lactons, das eine oder mehrere $C_1$- bis $C_4$-Alkylgruppen als Substituenten tragen kann, oder eines aliphatischen oder cycloaliphatischen Sulfons vornimmt und dabei ein Volumenverhältnis dieser Lösungsmittel zum Olefin II von 0,5 : 1 bis 100 : 1 einstellt.

Gemäß Ausführungsform (a) des erfindungsgemäßen Verfahrens nimmt man die Umsetzung in Gegenwart von wasserhaltigen Lösungsmitteln, insbesondere organischen Lösungsmitteln, vor, in denen Wasser löslich oder zumindest teilweise löslich ist, wobei die Löslichkeit unter den Reaktionsbedingungen nicht weniger als 1 Gew.% betragen sollte. Der Wassergehalt des gesamten Reaktionsgemisches liegt hierbei zwischen 0,1 und 20 Gew.%, vorzugsweise zwischen 0,1 und 10 Gew.%, wobei unter Reaktionsgemisch die Mischung aus dem Lösungsmittel mit dem Katalysator und dem Olefin II zu verstehen ist.

Der Anteil des organischen Lösungsmittels am Reaktionsgemisch beträgt normalerweise 40 bis 80 Gew.%, und für das Gewichtsverhältnis dieses Lösungsmittels zu Wasser empfiehlt sich ein Bereich von 5 : 1 bis 100 : 1, insbesondere von 10 : 1 bis 100 : 1. Außerdem ist es zweckmäßig, bei einem Volumenverhältnis des wasserhaltigen Lösungsmittels zum Olefin von 0,5 : 1 bis 100 : 1, vorzugsweise 0,5 : 1 bis 10 : 1, insbesondere 1 : 1 bis 10 : 1, zu arbeiten.

Als derartige organische Lösungsmittel kommen hierbei

- Ketone, beispielsweise Aceton, Diethylketon, Methylethylketon, Diisopropylketon, Methylisopropylketon, Acetophenon, Benzophenon, Cyclopentanon und Cyclohexanon,
- cyclische Ether wie Tetrahydrofuran und Dioxan,
- Carbonsäureester, beispielsweise Essigsäureethylester, Propionsäureethylester, Buttersäuremethylester und Valeriansäuremethylester, und
- hydroxylgruppenhaltige Carbonsäureester wie 3-Hydroxybuttersäureethylester und Isobuttersäure-3-hydroxy-2,2,4-trimethylpentylester

in Betracht. Besonders gute Ergebnisse erzielt man mit Ketonen.

Bei Ausführungsform (b) des erfindungsgemäßen Verfahrens wird das Olefin II in Gegenwart eines gegebenenfalls substituierten Lactons oder eines aliphatischen oder cycloaliphatischen Sulfons als Lösungsmittel hydroformyliert, wobei das Volumenverhältnis dieses Lösungsmittels zum Olefin 0,5 : 1 bis 100 : 1, vorzugsweise 0,5 : 1 bis 10 : 1, insbesondere 1 : 1 bis 10 : 1, beträgt. Dabei können weitere übliche organische Lösungsmittel wie Dioxan, Tetrahydrofuran, Cyclohexan, Toluol oder Xylole mit anwesend sein. Selbstverständlich können auch wasserhaltige Lactone oder Sulfone bei dieser Ausführungsform des Verfahrens verwendet werden - die Anwesenheit von Wasser in diesen Lösungsmitteln hat aber im allgemeinen keine weitere Verbesserung des Verfahrensergebnisses zur Folge. Die Bildung der Aldehyde I steigt mit wachsendem Anteil an Lacton bzw. Sulfon gegenüber den Olefinen II in der Reaktionsmischung an, man arbeitet deshalb zweckmäßigerweise mit einem Überschuß dieser Lösungsmittel.

Als Lactone kommen 5-, 6- und 7-Ring-Lactone, die gegebenenfalls eine oder mehrere $C_1$- bis $C_4$-Alkylgruppen als Substituenten tragen, in Betracht. Typische Vertreter hierbei sind γ-Butyrolacton, δ-Valerolacton und ε-Caprolacton. Am vorteilhaftesten können 5-Ring-Lactone wie vor allem γ-Butyrolacton, 4-Ethyl-γ-butyrolacton und 5-Methyl-γ-butyrolacton (γ-Valerolacton), daneben aber auch 3-Methyl-γ-butyrolacton, 4-Methyl-γ-butyrolacton, 3,4-Dimethyl-γ-butyrolacton, 4,5-Dimethyl-γ-butyrolacton, 5-Propyl-γ-butyrolacton oder 3,5-Diethyl-γ-butyrolacton eingesetzt werden.

Als Sulfone können Dimethylsulfon, Diethylsulfon, Tetramethylensulfon (Sulfolan) oder Pentamethylensulfon verwendet werden. Besonders gute Ergebnisse erzielt man mit Sulfolan.

Das erfindungsgemäße Verfahren eignet sich mit beiden Ausführungsformen besonders gut zur Herstellung von Pivalaldehyd (2,2-Dimethylpropionaldehyd) aus Isobuten. Daneben können nach diesem Verfahren aber auch andere Aldehyde der allgemeinen Formel I, beispielsweise 2,2-Dimethylbutyraldehyd, 2,2-Dimethylvaleraldehyd, 2,2-Dimethylisovaleraldehyd, 2,2-Dimethylhexanal, 2-Ethyl-2-methylbutyraldehyd, 2-Ethyl-2-methylvaleraldehyd, 2-Methyl-2-propylvaleraldehyd, 2-Ethyl-2-methylhexanal, 2-methyl-2-propylhexanal und 2-Butyl-2-methylhexanal, aus den entsprechenden Olefinen II vorteilhaft hergestellt werden.

Bis auf die erfindungswesentlichen Merkmale gemäß den Ausführungsformen (a) und (b) kann man die Hydroformylierung in an sich bekannter Weise diskontinuierlich oder kontinuierlich durchführen, d.h. bei Drücken von normalerweise 60 bis 330 bar, insbesondere 130 bis 280 bar, bei Temperaturen von üblicherweise 60 bis 200°C, insbesondere 80 bis 160°C, und bei einer Cobaltcarbonyl-Katalysatormenge von zweckmäßigerweise 0,1 bis 1 Gew.%, insbesondere 0,2 bis 0,5 Gew.% Cobalt, bezogen auf die Gesamtmenge des Reaktionsgemisches. Man verwendet in der Regel ein Synthesegas mit einem Volumenverhältnis von Kohlenmonoxid zu Wasserstoff von 0,5 : 1 bis 2 : 1, wobei ein äquimolares Volumenverhältnis von 1 : 1 besonders vorteilhaft ist.

Als Katalysatoren dienen üblicherweise im Reaktionsgemisch lösliche Cobaltcarbonyl-Komplexe. Der Katalysator kann beispielsweise als Carbonylverbindung wie $Co_2(CO)_8$, $Co_4(CO)_{12}$ und $HCo(CO)_4$ oder als Salz wie Cobaltacetat, Cobaltnitrat, Cobaltcarbonat und Cobalt-2-ethylhexanoat eingesetzt werden. Auch Cobaltoxide und metallisches Cobalt können verwendet werden. Die Cobaltverbindungen und das Cobalt bilden unter den Reaktionsbedingungen mit dem Synthesegas erst die katalytisch wirksamen Carbonyl-Komplexe.

Die Aufarbeitung des Reaktionsgemisches, das in der Regel neben dem Verfahrensprodukt I weitere Produkte wie zu I isomere Aldehyde, Reduktionsprodukte der gebildeten Aldehyde und Wasseranlagerungsprodukte an das eingesetzte Olefin II sowie nicht umgesetztes Olefin II enthalten kann, wird normalerweise wie üblich vorgenommen, wodurch sich nähere Angaben hierüber erübrigen.

Man erhält die 2,2-Dialkylpropionaldehyde I in deutlich höheren Ausbeuten, als es zuvor durch Hydroformylierung der entsprechenden Olefine II möglich war.

Beispiele 1 bis 9 und Vergleichsbeispiele A und B

Diskontinuierliche Hydroformylierung von Isobuten in wasserhaltigen Lösungsmitteln

Eine Lösung von 6,5 g Dicobaltoctacarbonyl in 400 g der in Tabelle 1 jeweils aufgeführten Lösungsmittel-Wasser-Mischung wurde mit 122 g flüssigem Isobuten versetzt. Durch Zugabe einer $CO/H_2$-Mischung im Vo-

lumenverhältnis von 1 : 1 wurde ein Druck von 100 bar eingestellt. Nach Erhöhung der Temperatur auf 110°C wurde durch weitere Zugabe dieser Gasmischung ein Druck von 280 bar eingestellt und über die Reaktionsdauer von 3 Stunden aufrechterhalten. Anschließend wurde das abgekühlte und entspannte Reaktionsgemisch gaschromatographisch analysiert.

Die Ergebnisse dieser Versuche sind der Tabelle 1 zu entnehmen.

Tabelle 1 (Beispiele 1 bis 9 und Vergleichsbeispiele A und B)

| Bsp. Nr. | Lösungsmittel | Wasser im Reaktionsgemisch [Gew.%] | Umsatz [%] | Ausbeute an PVA [%] | 3-MBA [%] | t-BuOH [%] |
|---|---|---|---|---|---|---|
| 1 | Dioxan | 5,7 | 91 | 8,3 | 64,8 | 12 |
| A | Dioxan | 0 | 75 | 2,3 | 50,2 | - |
| 2 | Isobuttersäure-3-hydroxy-2,2,4-trimethylpentylester | 3,0 | 94 | 5,1 | 60,0 | 4 |
| B | Isobuttersäure-3-hydroxy-2,2,4-trimethylpentylester | 0 | 89 | 2,0 | 57,0 | - |
| 3 | Aceton | 0,2 | 94 | 15,2 | 31,7 | 7 |
| 4 | Aceton | 5,7 | 97 | 26,7 | 41,0 | 26 |
| 5 | Diisopropylketon | 0,15 | 87 | 3,6 | 59,0 | - |
| 6 | Diisopropylketon | 2,8 | 91 | 7,9 | 56,5 | 8,5 |
| 7 | Diethylketon | 0,12 | 82 | 6,8 | 46,2 | 0,5 |
| 8 | Diethylketon | 2,8 | 89 | 12,6 | 44,5 | 1 |
| 9 | Diethylketon | 5,7 | 93 | 17,5 | 42,5 | 17 |

PVA: Pivalaldehyd, 3-MBA: 3-Methylbutyraldehyd, t-BuOH: tert.-Butanol

Beispiele 10 bis 14

Kontinuierliche Hydroformylierung von Isobuten in wasserhaltigen Lösungsmitteln

Analog den Beispielen 1 bis 9 bzw. den Vergleichsbeispielen A und B wurde die Hydroformylierung von Isobuten bei 280 bar und 110°C mit einer $CO/H_2$-Mischung im Volumenverhältnis von 1 : 1 innerhalb von 3 Stunden in Gegenwart der in Tabelle 2 aufgeführten Lösungsmittel-Wasser-Mischungen kontinuierlich durchgeführt. Dabei wurden stündlich 180 g der genannten Keton-Wasser-Mischung, die den gelösten $Co_2(CO)_8$-Katalysator enthielt, und und 61 g Isobuten durch den Reaktor gepumpt. Der Cobaltgehalt betrug 0,44 Gew.%, bezogen auf den Gesamtreaktionsstrom.

Tabelle 2 zeigt die gaschromatographisch ermittelten Ergebnisse dieser Versuche.

Tabelle 2 (Beispiele 10 bis 14)

| Bsp. Nr. | Lösungsmittel | Wasser im Reaktionsgemisch [Gew.%] | Umsatz [%] | Ausbeute an PVA [%] | 3-MBA [%] | t-BuOH [%] |
|---|---|---|---|---|---|---|
| 10 | Aceton | 0,15 | 85 | 12,5 | 34,6 | 3,1 |
| 11 | Aceton | 1,2 | 87 | 21,1 | 38,4 | 12,8 |
| 12 | Aceton | 5,5 | 92 | 22,5 | 29,6 | 12,4 |
| 13 | Diethylketon | 1,2 | 91 | 13,5 | 39,4 | 10,2 |
| 14 | Diethylketon | 5,4 | 88 | 19,5 | 29,3 | 16,1 |

PVA: Pivalaldehyd, 3-MBA: 3-Methylbutyraldehyd, t-BuOH: tert.-Butanol

Beispiele 15 bis 19 und Vergleichsbeispiel C

Hydroformylierung von Isobuten in Gegenwart eines Sulfons oder Lactons

Eine Lösung von 7,6 g Dicobaltoctacarbonyl in 400 g des in Tabelle 3 jeweils aufgeführten Lösungsmittels wurde mit 122 g flüssigem Isobuten (entsprechend 200 ml) versetzt. Durch Zugabe einer $CO/H_2$-Mischung im Volumenverhältnis von 1 : 1 wurde ein Druck von 100 bar eingestellt. Nach Erhöhung der Temperatur auf 110°C wurde durch weitere Zugabe dieser Gasmischung ein Druck von 280 bar eingestellt und über die Reaktionsdauer von 3 Stunden aufrechterhalten. Anschließend wurde das abgekühlte und entspannte Reaktionsgemisch gaschromatographisch analysiert.

Die Ergebnisse dieser Versuche sind der Tabelle 3 zu entnehmen. Der Umsatz an Isobuten lag bei den Beispielen 15 bis 19 jeweils über 90 %.

Tabelle 3 (Beispiele 15 bis 19 und Vergleichsbeispiel C)

| Beispiel Nr. | Lösungsmittel | Volumenverhältnis Lösungsmittel zu Isobuten | Ausbeute an PVA [%] | 3-MBA [%] |
|---|---|---|---|---|
| 15 | Sulfolan | 1,57 : 1 | 27,8 | 2 |
| 16 | γ-Butyrolacton | 1,81 : 1 | 25,2 | 6 |
| 17 | 5-Methyl-γ-butyrolacton | 1,90 : 1 | 37,5 | 6 |
| 18 | 4-Ethyl-γ-butyrolacton | 1,90 : 1 | 25,0 | 9 |
| 19 | ε-Caprolacton | 1,94 : 1 | 17,2 | 3 |
| C | Tetrahydrofuran | 2,25 : 1 | 6,4 | 74,2 |

PVA: Pivalaldehyd, 3-MBA: 3-Methylbutyraldehyd

Beispiele 20 bis 22 und Vergleichsbeispiele D und E

Hydroformylierung von Isobuten in Gegenwart von Sulfolan und Toluol

Analog den Beispielen 15 bis 19 bzw. dem Vergleichsbeispiel C wurde die Hydroformylierung von Isobuten bei 280 bar und 110°C mit einer $CO/H_2$-Mischung im Volumenverhältnis von 1 : 1 innerhalb von 3 Stunden in Gegenwart von Sulfolan und zusätzlich von wechselnden Mengen an Toluol durchgeführt. Die

Reaktorfüllmenge von 600 ml Reaktionsgemisch, bestehend aus 122 g flüssigem Isobuten (entsprechend 200 ml) und 400 ml Lösungsmittel, wobei das Volumen des Katalysators vernachlässigt wurde, wurde dabei konstant gehalten und Sulfolan zunehmend durch Toluol ersetzt. Die Menge des Katalysators $Co_2(CO)_8$ betrug bei den Beispielen 20 bis 22 und den Vergleichsbeispielen D und E jeweils 4,6 g.

Tabelle 4 zeigt Einzelheiten und die gaschromatographisch ermittelten Ergebnisse dieser Versuche. Bei Sulfolan/Isobuten-Verhältnissen von kleiner als 0,5 : 1 wurden lediglich die Ausbeuten an Pivalaldehyd erzielt, die der Stand der Technik angibt.

Tabelle 4 (Beispiele 20 bis 22 und Vergleichsbeispiel D,E)

| Beispiel Nr. | Volumen an Sulfolan [ml] | Volumen an Toluol [ml] | Volumenverhältnis Sulfolan zu Isobuten | Ausbeute an PVA [%] | 3-MBA [%] |
|---|---|---|---|---|---|
| 20 | 400 | 0 | 2 : 1 | 19 | 2 |
| 21 | 200 | 200 | 1 : 1 | 17 | 35 |
| 22 | 100 | 300 | 0,5 : 1 | 8 | 56 |
| D | 50 | 350 | 0,25 : 1 | 4 | 62 |
| E | 0 | 400 | - | 2 | 54 |

PVA: Pivalaldehyd, 3-MBA: 3-Methylbutyraldehyd

## Patentansprüche

1. Verfahren zur Herstellung von 2,2-Dialkylpropionaldehyden der allgemeinen Formel I

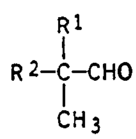

I

in der $R^1$ und $R^2$ für $C_1$- bis $C_4$-Alkylgruppen stehen, durch Hydroformylierung von 1,1-Dialkylethylenen der allgemeinen Formel II

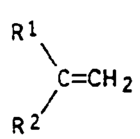

II

mit Cobaltcarbonyl-Komplexen, dadurch gekennzeichnet, daß man
(a) die Umsetzung in Gegenwart von wasserhaltigen Lösungsmitteln durchführt, in denen Wasser löslich oder zumindest teilweise löslich ist, wobei der Wassergehalt des Reaktionsgemisches zwischen 0,1 und 20 Gew.% liegt, oder
(b) die Umsetzung in Gegenwart eines Lactons, das eine oder mehrere $C_1$- bis $C_4$-Alkylgruppen als Substituenten tragen kann, oder eines aliphatischen oder cycloaliphatischen Sulfons vornimmt und dabei ein Volumenverhältnis dieser Lösungsmittel zum Olefin II von 0,5 : 1 bis 100 : 1 einstellt.

2. Verfahren zur Herstellung von 2,2-Dialkylpropionaldehyden nach Anspruch 1, dadurch gekennzeichnet, daß man bei der Ausführungsform (a) die Umsetzung bei einem Wassergehalt des Reaktionsgemisches

von 0,1 bis 10 Gew.% durchführt.

3. Verfahren zur Herstellung von 2,2-Dialkylpropionaldehyden nach Anspruch 1, dadurch gekennzeichnet, daß man bei der Ausführungsform (b) die Umsetzung bei einem Volumenverhältnis des Lactons oder Sulfons zu II von 0,5 : 1 bis 10 : 1 vornimmt.

## Claims

1. A process for the preparation of 2,2-dialkylpropionaldehydes of the general formula I

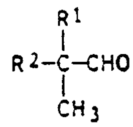

I

where $R^1$ and $R^2$ are each $C_1$-$C_4$-alkyl, by hydroformylation of 1,1-dialkylethylenes of the general formula II

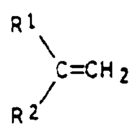

II

with cobalt carbonyl complexes, which comprises
(a) carrying out the reaction in the presence of water-containing solvents in which water is soluble or at least partially soluble, with the water content of the reaction mixture being from 0.1 to 20 % by weight, or
(b) carrying out the reaction in the presence of a lactone which can have one or more $C_1$-$C_4$-alkyl substituents, or of an aliphatic or cycloaliphatic sulfone, and moreover adjusting the ratio by volume of these solvents to the olefin II to from 0.5:1 to 100:1.

2. A process for the preparation of 2,2-dialkylpropionaldehydes as claimed in claim 1, wherein the reaction in embodiment (a) is carried out with the reaction mixture containing from 0.1 to 10 % by weight of water.

3. A process for the preparation of 2,2-dialkylpropionaldehydes as claimed in claim 1, wherein the reaction in embodiment (b) is carried out with a ratio of the lactone or sulfone to II of 0.5:1 to 10:1 by volume.

## Revendications

1. Procédé de préparation de 2,2-dialkylpropionaldéhydes de formule générale I

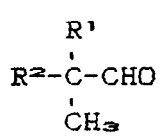

I

dans laquelle $R^1$ et $R^2$ sont mis pour des groupements alkyle en $C_1$ à $C_4$, par hydroformylation de 1,1-diallyléthylènes de formule générale II

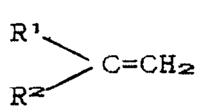

II

avec des complexes cobalt-carbonyle, caractérisé en ce que

(a) on conduit la réaction en présence de solvants hydratés dans lesquels l'eau est soluble ou au moins partiellement soluble, la teneur en eau du mélange réactionnel se situant entre 0,1 et 20% en poids, ou

(b) on effectue la réaction en présence d'une lactone, qui peut porter un ou plusieurs groupements allyle en $C_1$ à $C_4$ en tant que substituants, ou en présence d'une sulfone aliphatique ou cycloaliphatique et on règle alors un rapport volumique de ces solvants à l'oléfine II de 0,5:1 à 100:1.

**2.** Procédé de préparation de 2, 2-dialkylpropionaldéhydes selon la revendication 1, caractérisé en ce que, dans la forme de réalisation (a), on conduit la réaction avec une teneur en eau du mélange réactionnel de 0,1 à 10% en poids.

**3.** Procédé de préparation de 2,2-dialkylpropionaldéhydes selon la revendication 1, caractérisé en ce que, dans la forme de réalisation (b), on effectue la réaction avec un rapport volumique de la lactone ou de la sulfone à II de 0,5:1 à 10:1.